Europäisches Patentamt

**European Patent Office**

Office européen des brevets

Numéro de publication: **0 276 331**
**A1**

## DEMANDE DE BREVET EUROPEEN

Numéro de dépôt: **86402886.5**

Int. Cl.⁴ **A61F 2/16**

Date de dépôt: **22.12.86**

Date de publication de la demande:
**03.08.88 Bulletin 88/31**

Etats contractants désignés:
**BE CH DE FR GB LI NL SE**

Demandeur: **Meur, Guy**
**86 rue Tenbosch**
**B-1050 Bruxelles(BE)**

Inventeur: **Meur, Guy**
**86 rue Tenbosch**
**B-1050 Bruxelles(BE)**

Mandataire: **Viard, Jean**
**Cabinet VIARD 28 bis, avenue Mozart**
**F-75016 Paris(FR)**

Implant intraoculaire de correction de l'aphakie.

Implant intraoculaire de correction de l'aphakie constitué par une lentille centrale, la lentille (1) étant entourée par au moins deux échelons de Fresnel (4, 5, 6) ayant la même puissance réfractive que celle de la lentille (1).

FIG.2

La présente invention a pour objet un implant intraoculaire cristallin artificiel destiné à pallier l'absence de cristallin due notamment à une opération intra ou extra-capsulaire de la cataracte.

Le cristallin naturel chez l'homme est une structure transparente dont le diamètre est de l'ordre de 9mm pour une épaisseur de 5mm envion, de forme générale lenticulaire, suspendue derrière l'iris par des fibres oculaires qui relient le cristallin au corps ciliaire. La cataracte consiste en une opacification du cristallin, ou de l'une des capsules antérieure ou postérieure qui constitue le sac du cristallin. L'opération de la cataracte consiste en l'ablation du cristallin et est désignée comme "intracapsulaire" lorsque la capsule postérieure est retirée en même temps que le cristallin, et comme "extracapsulaire" lorsque la capsule antérieure est extraite avec le cristallin alors que la capsule postérieure est laissée à l'intérieur de l'oeil.

Ce type d'implant a été proposé dès 1949 par RIDLEY en vue d'éviter à l'opéré le port permanent de lunettes à verres très épais. Depuis cette date, de nombreux implants ont été proposés et l'on peut citer notamment les implants décrits dans les brevets US-A-4 340 979 (KELMAN), EP-A-O 053 384 (FAULKNER), US-A-4 244 060 (HOFFER), WO-84/00883 (CASTELMAN).

Un implant de ce type comprend en général une lentille, dont la puissance, et par suite la géométrie, est adaptée au cas particulier de chaque patient, prenant appui au moyen d'anses souples ou haptiques dans une des cavités de l'oeil antérieure ou postérieure par rapport au plan de la pupille. C'est ainsi que dans les implants connus, les haptiques prennent appui dans l'angle iridocornéen, sur le sulcus ciliaire ou dans le sac capsulaire.

Un élément essentiel d'un bon résultat obtenu avec un tel implant est l'obtention d'un centrage correct de manière à ce que l'image se forme exactement sur la rétine. En particulier, il est impératif que jamais le bord de la lentille n'apparaisse dans l'aire pupillaire, ce qui entraînerait une double réfraction avec diplopie monoculaire perturbant gravement la vision.

Afin de remédier à cet inconvénient, on a déjà proposé d'augmenter le diamètre de l'optique, habituellement de l'ordre de 5 à 6mm, pour porter celui-ci jusqu'à 9mm. Mais cette augmentation de diamètre entraîne une augmentation de poids proportionnelle au cube du diamètre.

Or, le poids de l'implant est toujours un facteur défavorable. En effet, l'oeil est soumis en permanence à différents mouvements de rotation. Par suite, la lentille est soumise à des accélérations et à des efforts centrifuges qui engendrent, sur les tissus oculaires avec lesquels elle est en contact, des pressions d'autant plus fortes que le poids de la prothèse est élevé.

Selon la présente invention, l'implant intraoculaire de correction de l'aphakie est caractérisé en ce que l'optique est constituée par une lentille centrale entourée d'au moins deux échelons de Fresnel ayant la même puissance réfractive que celle de la lentille centrale.

Ainsi, il est possible d'obtenir le même résultat qu'avec une lentille dont le diamètre est relativement grand, en réalisant un gain de poids notable grâce à la formation d'échelons de Fresnel. De plus, la présence de ces échelons reporte une partie du poids sur la périphérie, ce qui est favorable à un meilleur équilibre de l'ensemble.

Selon une autre caractéristique de l'invention, l'optique est monobloc et est obtenue, par moulage ou détourage d'une matière transparente biocompatible souple ou rigide telle que, par exemple, du polyméthylmétacrylate PMMA, du silicone, du verre, etc...

Selon le résultat recherché, la lentille centrale peut être soit plan convexe à convexité tournée vers l'avant ou vers l'arrière de l'oeil, biconvexe, ou ménisque, son diamètre étant compris par exemple entre 4,5 et 6 mm.

L'optique peut être fixée dans la chambre antérieure ou dans la chambre postérieure, soit par des haptiques comme connu en soi, soit sans haptique. Dans ce dernier cas (implant disque), le diamètre total de l'optique doit être égal ou supérieur à 9 mm.

D'autres caractéristiques et avantages de la présente invention apparaîtront au cours de la description qui va suivre de modes particuliers de réalisation, donnés uniquement à titre d'exemples non limitatifs, en regard des dessins qui représentent :

- La figure 1, une vue de profil d'un implant selon l'invention, à deux échelons de Fresnel ;

- la figure 2, dans les mêmes conditions, un second implant présentant trois échelons de Fresnel ;

- la figure 3, une vue par l'arrière d'un implant selon l'invention, correspondante au profil de la figure 2.

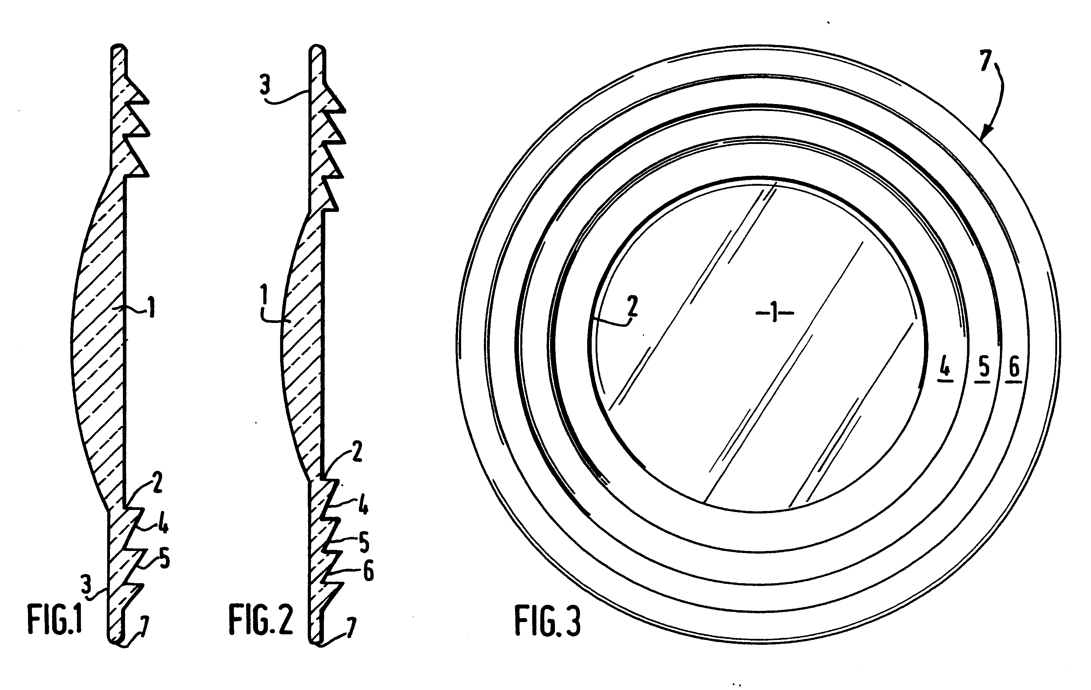

Sur la figure 1, on voit que l'implant se compose d'une lentille centrale 1 qui dans l'exemple représenté est une lentille plan convexe dont le diamètre est par exemple de 5mm. Le bord 2 de la lentille se prolonge par une zone circulaire en forme d'anneau 3. Dans la zone 3 sont constitués deux échelons de Fresnel respectivement 4 et 5 présentant la même puissance réfractive que celle de la lentille 1 et dont les bords peuvent être légèrement arrondis pour ne pas léser la capsule postérieure. Le bord extérieur 7 de l'implant, qui dans l'exemple représenté est un implant disque utilisé sans haptique, présente un diamètre total de 9.2mm.

La figure 2 représente un autre implant dont la seule différence avec l'implant de la figure 1 réside en ce que la zone 3 présente trois échelons de Fresnel respectivement 4, 5 et 6. Il est ainsi possible de voir des échelons d'épaisseur moins élevée que dans le cas précédent. Dans les deux exemples représentés, le bord 7 de la couronne 3 est bien entendu arrondi, de manière à ne pas provoquer d'irritation sur les tissus contre lesquels il vient en appui.

La figure 3 représente en vue de face un implant tel que celui qui a été représenté sur la figure 2. On y retrouve la lentille 1 et la zone circulaire 3 entourant la lentille. Comme cela apparaît sur la figure, on distingue dans la zone 3 trois échelons ou rainures 4, 5 et 6 constituant les échelons de Freshnel.

La présence de ces échelons permet un gain notable de poids, par rapport à une lentille de même diamètre constituant un implant disque.

Il va de soi que de nombreuses variantes peuvent être introduites, notamment par substitution de moyens techniquement équivalents sans sortir pour celà du cadre de l'invention.

**Revendications**

1. Implant intraoculaire de correction de l'aphakie, constitué par une lentille centrale, caractérisé en ce que la lentille (1) est entourée par au moins deux échelons de Freshnel (4, 5, 6) ayant la même puissance réfractive que celle de la lentille (1).

2. Implant selon la revendication 1, caractérisé en ce qu'il est monobloc et obtenu par moulage ou détourage d'une matière transparente bio-compatible souple ou rigide.

FIG.1

FIG.2

FIG.3

0 276 331

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | EP-A-0 180 887 (INPROHOLD) * Revendications 1-4; figures 1-8; résumé * | 1,2 | A 61 F 2/16 |
| | ----- | | |

DOMAINES TECHNIQUES RECHERCHES (Int. Cl.4)

A 61 F

Le présent rapport de recherche a été établi pour toutes les revendications

| Lieu de la recherche | Date d'achèvement de la recherche | Examinateur |
|---|---|---|
| LA HAYE | 03-07-1987 | ARGENTINI A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82